# EUROPEAN PATENT SPECIFICATION

(11) **EP 2 777 718 B1**
(45) Date of publication and mention of the grant of the patent: **12.08.2020**
(21) Application number: 13159273.5
(22) Date of filing: 14.03.2013
(51) Int. Cl.: A61L 9/12, B60H 3/00

(54) **Fragrance diffuser with cartridge**
Duftstoffspender mit Kartusche
Dispositif d'odorisation avec cartouche

(43) Date of publication of application: 17.09.2014
(73) Proprietor: Valeo Klimasysteme GmbH, 96476 Bad Rodach (DE)
(72) Inventor: Feuillard, Vincent, 78320 Le Mesnil-Saint-Denis (FR); Lauer, Oliver, 96237 Ebersdorf (DE); Albrecht, Sebastian, 96450 Coburg (DE)
(74) Representative: Prinz & Partner mbB

(56) References cited:
- GB-A- 2 462 274
- JP-A- H0 337 069
- US-A1- 2005 220 664
- US-A1- 2008 169 306

## Description

The invention relates to a cartridge for a fragrance diffuser. The invention further relates to a fragrance diffuser. Furthermore, the invention relates to a method for transferring a fragrance cartridge from an initial condition into a condition of use.

Fragrance diffusers are used to enhance the air quality in a room or the passenger cabin of a vehicle. For this purpose a fragrance containing liquid is evaporated so that the gaseous fragrance can diffuse within the whole room or passenger cabin. Hermetically sealed cartridges filled with the fragrance containing liquid provide the fragrance and are inserted into the fragrance diffuser. Before or during the insertion the cartridges are unsealed so that the fragrance containing liquid can be diffused. In order to close the inserted cartridge when no diffusion of the fragrance is desired, the fragrance diffuser is provided with a cover to close the cartridge. However, known fragrance diffusers cannot close the cartridge hermetically, leading to a constant diffusion of the fragrance containing liquid. Especially when the car is parked and exposed to high temperatures the cartridge leaks, which results in a very strong scent in the cabin and also results in a reduced lifetime of the cartridge.

US 2005/0220664 A1 shows a cartridge for a fragrance diffuser comprising a body, wherein an aroma substance is provided in the interior of the body. The aroma substance can be dispensed via a metering device comprising a plunger. Further, a cap is provided that can be screwed onto the cartridge in order to protect the plunger.

US 2008/0169306 A1 discloses a fragrance diffuser comprising a movable piston with a wick that can be lowered into a cartridge through an opening in the cartridge.

GB 2462274 A shows a fragrance diffuser with several fragrance cartridges. The cartridges comprise a body and a rigid wick which extends through a lid in the body, wherein the lid seals the interior of the cartridge. The part of the wick that extends externally of the body can be covered by a sliding cover which can be securely locked to the lid.

JP H03 37 069 A discloses a fragrance diffuser comprising a body, in which an aroma substance is provided, and a pull-off lid which seals the body and prevents the aroma substance from leaking prior to first use of the fragrance diffuser.

The object of the present invention is to provide a fragrance diffuser with suitable cartridges that show no leakage when the diffusion of the fragrance containing liquid is undesired.

For this purpose, the invention provides a cartridge for a fragrance diffuser, in particular for vehicles, according to claim 1. Thus, an easy and reliable way of hermetically closing the cartridge is realized. This way a cartridge is provided which is airtight not only in a position in which the cover of the cartridge is securely locked to the body but also in a position that allows the cover to be easily opened without the need for much force. Thus, the cartridge can be transferred from a closed position, which is airtight, into an open position and vice versa by means of the fragrance diffuser, allowing the fragrance diffuser to hermetically seal the cartridge after use. By means of the element of the body, the body of the cartridge can be vertically fixed in position in the fragrance diffuser. Further, the element of the cover allows the housing of the fragrance diffuser to guide the opening movement of the cover.

Preferably, a latch mechanism is provided which is adapted for locking the cover to the body, hereby achieving a locking mechanism which is cost efficient in fabrication.

According to a further embodiment of the invention, the element of the body is a projection for cooperation with a housing of a fragrance diffuser.

Preferably, the element of the cover is a projection for cooperation with a housing of a fragrance diffuser.

Furthermore, the cover can be provided with an engagement element for cooperation with an actuating device thus providing a means for the fragrance diffuser to actuate the cover.

The invention further provides a fragrance diffuser according to claim 6, in particular for vehicles, having a receiving space for a cartridge according to the invention and an actuating device for opening the cover of the cartridge, wherein the actuating device comprises a device for transferring the cartridge from its locked condition or storage condition into its condition ready for use within the diffuser. The actuating device comprises two cooperation elements for engaging with the body and the cover, respectively. Thus, no external manipulation of the cartridge is necessary and the cartridge can be closed by the actuating device after usage. Further, the fragrance diffuser is able to guide the movement of the cover relative to the movement of the body.

The actuating device can comprise a stepper motor. This stepper motor provides simple but precise means to open the cover of the cartridge.

Preferably, the actuating device comprises an actuating element for cooperation with the cover of the cartridge. This way the movement of the actuating device is easily transferred into a movement of the cover.

The actuating element can be a pin which is a cost efficient way of fabricating an actuating element.

The cooperation elements can be formed as grooves with which elements formed on the cartridge body and the cartridge can engage, which is a simple and cost efficient way of designing the cooperation elements.

The groove provided for cooperation with the body can extend generally straight so that the cartridge can be easily inserted into the fragrance diffuser.

According to a preferred embodiment, the groove provided for cooperation with the cover extends, viewed in the insertion direction of the cartridge, from a starting level away from the groove provided for cooperation with the body and the back towards said groove, but ends at a level which is spaced further apart from said groove than the starting level. This geometry provides a simple means for transferring the cartridge from an initial condition to a condition ready for use.

Preferably, the groove provided for cooperation with the cover is provided with a lifting section which extends approximately perpendicularly away from the groove provided for cooperation with the body. This lifting section can guide the cover when it is opened.

The invention further provides a method according to claim 11 for transferring a fragrance cartridge from an initial condition into a condition of use.

Further features and advantages of the invention will be apparent from the following description of the embodiments and the attached drawings to which reference is made and in which:
- Figure 1 is a side view of the cartridge according to the invention,
- Figure 2 is a perspective view of the cartridge of Figure 1,
- Figures 3a) and b) show three different embodiments of the cross section of the cartridge according to Figure 1,
- Figure 4 is a perspective view of a fragrance diffuser according to the invention,
- Figure 5 is a perspective view of a side wall of a housing of the fragrance diffuser of Figure 4, and
- Figure 6 is an enlarged view of a grooved section of the side wall of Figure 5.

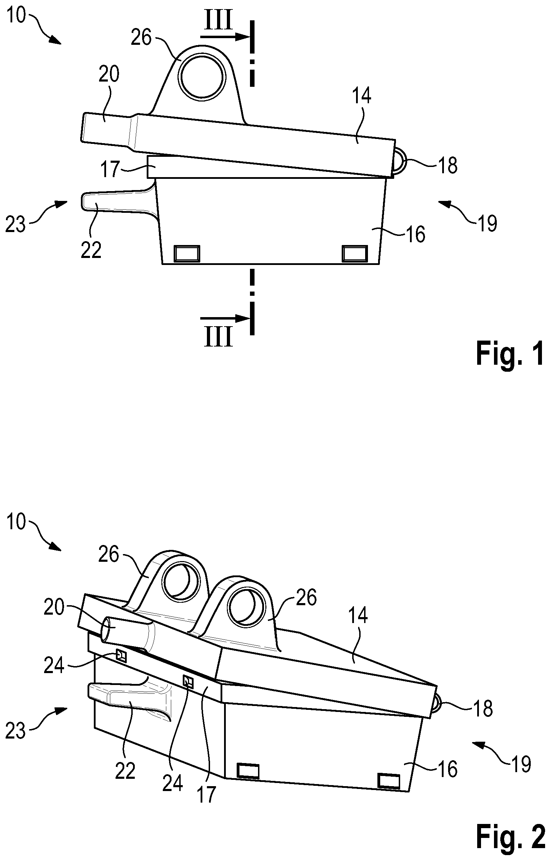

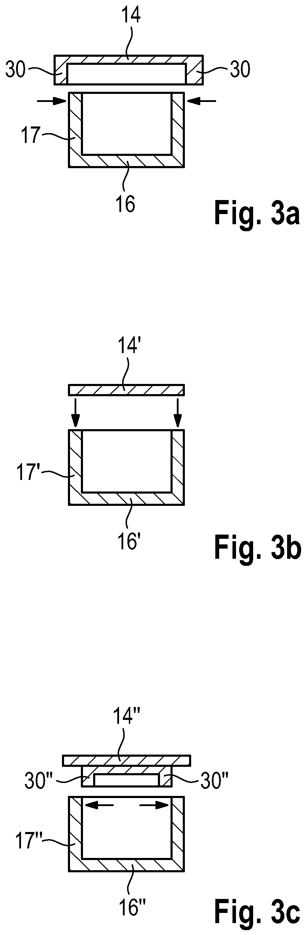

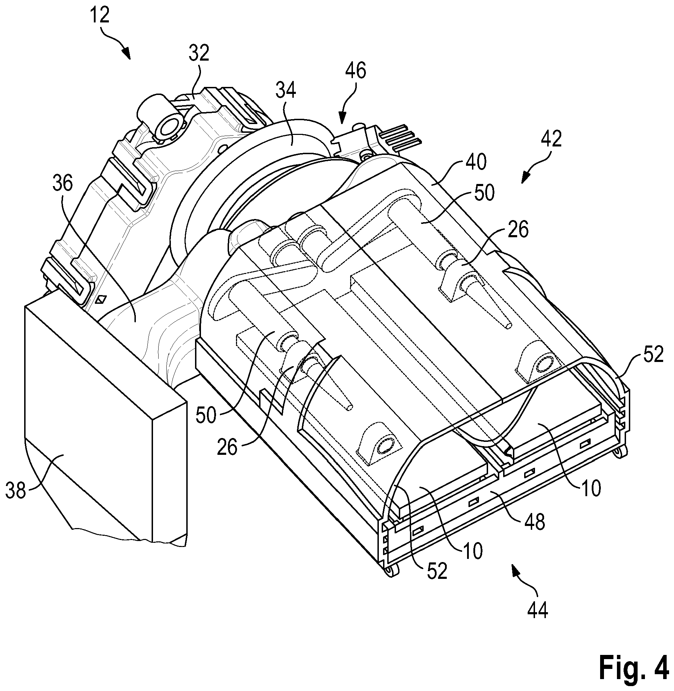

Figure 1 and Figure 2 show a cartridge 10 for a fragrance diffuser 12 (Fig. 4) with a cover 14 and a body 16. The cover 14 has a U-shaped cross section (comp. Fig. 3a)) opened towards the body 16. The body 16 is box-shaped and open towards cover 14, wherein the upper edge of body 16 forms a rim 17. Cover 14 and body 16 are connected via a film hinge 18 at the back side 19 of the cartridge 10 and are both provided with an element 20, 22 for cooperation with a housing of a fragrance diffuser, respectively. Preferably, the elements 20, 22 are projections extending in the same direction from the front side 23 of the cartridge 10.

On the front side 23 recesses 24 are formed on the outer side of rim 17 of body 16. Associated tongues (not shown) that fit into the recesses 24 are formed on the inner side of the edge of the cover 14. Recesses 24 and the tongues form a locking mechanism of the cartridge 10.

On the outer upper side of the cover 14 engagement elements 26 are formed as upstanding lugs. The engagement elements 26 each include a hole extending parallel to the front side 23 of the cartridge 10.

Figure 3a) shows a cross sectional view of the cartridge 10. The cover 14 has a U-shaped cross section with legs 30 which sides can engage on the outer side of the body 16. The arrows indicate the position of the locking mechanism of cartridge 10 whose tongues can either be fixed to the body 16 or the cover 14. If fixed to the body 16, the tongues are arranged at the outer side of the rim 17 of the body 16. If arranged on the cover 14, the tongues are fixed to the inner side of the legs 30 of the cover 14.

A seal (not shown) is provided on the horizontal surface of the rim 17 which is fixed to the body 16, for example by gluing or molding.

The cover 14 of the cartridge 10 can assume different positions relative to the body 16: a locked, a closed and an open position. The open position is shown in Figures 1 and 2, to which reference is made.

In the locked position the cover 14 abuts the rim 17 of body 16. In this position the seal closes the gap between the outer side of rim 17 of the body 16 and the inner side of the legs 30 of the cover 14 thus closing the interior of the body hermetically.

The tongues of cover 14 engage with the recesses 24 of body 16 locking the cover 14 securely to the body 16. In this locked position the cartridge 10 can be securely handled and stored outside the fragrance diffuser.

In its closed position the cover 14 is slightly lifted relative to its locking position. The seal still ensures that the interior of the body 16 is kept airtight. At the same time the tongues of cover 14 no longer engage with the recesses 24 of body 16. Therefore, the cover 14 and with that the seal can easily be opened without applying much force. This enables a comparatively light actuating mechanism of a fragrance diffuser to open the cover 14.

To reach the open position, the cover 14 is lifted further so that the seal cannot prevent the exchange of air between the interior of the cartridge 10 and the environment anymore. In the open position the film hinge 18 ensures the correct positioning of cover 14 to body 16 at all times.

The Figure 3b) shows a different embodiment of the cross section of the cartridge 10. In the embodiment shown in Figure 3b) the cover 14' has a double T-shaped cross section wherein the legs 30' of the T-shaped cover 14' fit to the inner sides of rim 17' of body 16'. The tongues of the locking mechanism are located in this embodiment either at the inner side of the rim 17' or the outer side of the legs 30' of the cover 14'.

Figure 4 shows the fragrance diffuser 12 with two inserted cartridges 10. The fragrance diffuser comprises a stepper motor 32 connected to a cam drive 34, an air outlet channel 36 connected to a blower 38 and a housing 40 of a receiving space 42. The housing has an air inlet side 44 and an air outlet side 46 and is open at both of these sides 44, 46. The air outlet channel 36 is connected with the opening of the air outlet side 46 of housing 40.

Two cartridges 10 can be located inside the housing 40 of the receiving space 42 with their back sides 19 facing each other. Although two cartridges 10 are intended for housing 40, for the ease of description only one of the two identical cartridges 10 and opening mechanisms is described. The second cartridge 10 and its according opening mechanism work accordingly.

The cartridge 10 sits in a drawer 48 which is slideably mounted inside the housing 40. An actuating element 50, in particular a pin, engages in one of the engagement elements 26 on top of cover 14. The actuating element 50 is mechanically connected to the cam drive 34 in a way that the stepper motor 32 can drive the actuating element 50 via the cam drive 34 such that it performs arcuate movements.

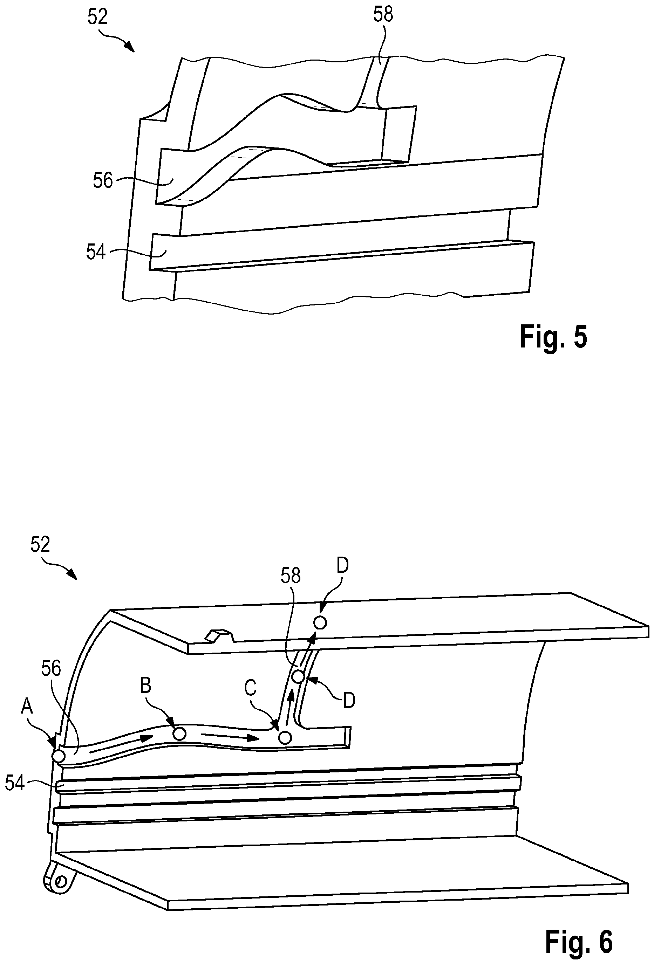

The housing 40 comprises a housing wall 52 which has grooves on its inner side. The inner side of the housing wall 52 can be seen in Figures 5 and 6. A groove 54 is provided for cooperation with element 22 of the body 16 and extends generally straight along the housing wall 52. A second groove 56 is formed above the groove 54 and is designed for cooperation with the element 20 of the cover 14. Starting on the air inlet side 44 of housing 40, which is the left edge of the housing 40, with respect to Figure 5, the groove 56 extends generally towards the air outlet side 46 of housing 40. Firstly, the groove 56 rises until it reaches a peak, before it falls to a level which is higher than its level at the edge of the housing 40. A lifting section 58 extends as a groove from and perpendicular to groove 54. As the housing wall 52 has an arcuate section, the lifting section 58 also describes an arc. Both of the groove 54 and the groove 56 are open at the air inlet side 44 of housing 40.

A cartridge 10 is inserted sideways into the fragrance diffuser 12 using the drawer 48 and through the air inlet side 44 of housing 40. During the insertion the element 20 and the element 22 engage with the groove 56 and the groove 54, respectively. The position of the first engagement of element 20 is indicated in Figure 5 with the letter A.

When the cartridge 10 is moved towards the air outlet side 46 the contour of groove 56 causes the element 20 and with it the cover 14 to rise until the peak of the contour of groove 56 is reached (position B). During this movement the cartridge10 has been transferred from its locked position to its open position.

A further insertion of the cartridge 10 into the housing 40 closes its cover 14 due to the falling slope of groove 56. In this position (position C) the cartridge is in its closed position, meaning that it is airtight but unlocked.

Furthermore, the cartridge 10 is now fully inserted into the housing 40. During the insertion one of the engagement elements 26 has engaged with the actuating element 50. In the shown embodiment, one of the holes in the engagement element 26 has been engaged by the actuating element 50 in form of a pin. Thus, the cover 14 can be lifted using the stepper motor 32 connected to the actuating element 50. The arcuate movement of actuating element 50 opens the cover 14 of the cartridge 10, and element 20 enters the lifting section 58 until the cartridge 10 has been opened to the desired extent (position D). The cover 14 is now in its open position in which the fragrance containing liquid comes in contact with the environment.

The fragrance diffuser 12 is now able to diffuse the fragrance containing liquid in the cartridge 10, possibly using a wick, and distributing the gaseous fragrance. This is achieved by the action of the blower 38 which creates a stream of air entering the fragrance diffuser 12 at the air inlet side 46 of housing 40 and passing the open cartridges 10, thereby diffusing parts of the fragrance containing liquid. Through the air outlet side 46 of housing 40 the air is moved through channel 36 and through blower 38 into the passenger cabin.

If the fragrance diffuser 12 is turned off, the cover 14 is closed by the actuating element 50 and the stepper motor 32 until element 20 reaches position C. Then the cover 14 is in its closed position providing an airtight seal of cartridge 10 and therefore preventing undesired leakage of fragrance.

Furthermore the cartridge 10 can be extracted from the housing 12 when the fragrance diffuser 12 is turned off. This is done in the reverse manner as the insertion of the cartridge 10. The elements 20, 22 slide in the grooves 56, 54 to the air inlet side 44, thereby moving the cover 14 towards the body 16. When the elements 20, 22 reach the air inlet side 44, the cover 14 has been lowered to an extend that the tongues of the cover 14 engage in the recesses 24 of the body 16. Thus the cover 14 is transferred to its locked position and the cartridge 10 is hermetically sealed and can safely be stored.

In the locked position, the amount of air trapped inside the cartridge 10 is very small, avoiding a peak smell when inserting the cartridge 10 into the fragrance diffuser 12 once more. This leads to an increased lifetime of the cartridge 10.

## Claims

1. Cartridge (10) for a fragrance diffuser (12), in particular for vehicles, comprising a body (16) and a cover (14) attached to the body (16), the cover (14) being able to assume a locked position in which it is securely locked to the body (16) such that the interior of the cartridge (10) is sealed, a closed position in which it cooperates with the body (16) such that the interior of the cartridge (10) is still sealed but unlocked, and an open position in which access to the interior of the cartridge (10) is possible, wherein the body (16) is provided with an element (22) for cooperation with a housing (40) of the fragrance diffuser (12) and wherein the cover (14) is provided with an element (20) for cooperation with the housing (40), wherein a seal is provided which seals the interior of the cartridge (10) both in the locked and the closed position of the cover (14), **characterized in that** a film hinge (18) is provided between the body (16) and the cover (14).

2. Cartridge according to claim 1, wherein a latch mechanism is provided which is adapted for locking the cover (14) to the body (16).

3. Cartridge according to any of the preceding claims, wherein the element (22) of the body (16) is a projection for cooperation with the housing (40) of a fragrance diffuser (12).

4. Cartridge according to any of the preceding claims, wherein the element (20) of the cover (14) is a projection for cooperation with the housing (40) of a fragrance diffuser (12).

5. Cartridge according to any of the preceding claims, wherein the cover (14) is provided with an engagement element (26) for cooperation with an actuating device.

6. Fragrance diffuser, in particular for vehicles, comprising a cartridge (10) as defined in any of the preceding claims and having a receiving space (42) for the cartridge (10) and an actuating device for opening the cover (14) of the cartridge (10), wherein the actuating device comprises a device for transferring the cartridge (10) from its locked condition into its closed condition ready for use within the fragrance diffuser (12), **characterized in that** the actuating device comprises two cooperation elements for engaging with the cover (14) and the body (16) of the cartridge (10), respectively.

7. Fragrance diffuser according to claim 6, wherein the actuating device comprises a stepper motor (32).

8. Fragrance diffuser according to claim 6 or 7, wherein the actuating device comprises an actuating element (50) for cooperation with the cover (14) of the cartridge (10).

9. Fragrance diffuser according to claim 8, wherein the actuating element (50) is a pin.

10. Fragrance diffuser according to any of the claims 6 to 9, wherein the cooperation elements are formed as grooves (54, 56) with which elements formed on the cartridge body (16) and the cartridge cover (14) can engage.

11. Method for transferring a fragrance cartridge (10) as defined in any one of the claims 1 to 5 from an initial condition into a condition of use, wherein the cartridge (10) having a body (16) and a cover (14) is inserted into a fragrance diffuser (12), and during the insertion the cover (14) being automatically transferred from a locked position in which the interior of the cartridge (10) is hermetically sealed by means of the cover (14), into a closed position in which the cover (14) still seals the interior of the body (16) but is no longer locked to the body (16).

## Patentansprüche

1. Kartusche (10) für einen Duftstoffdiffusor (12), insbesondere für Fahrzeuge, mit einem Körper (16) und einem am Körper (16) befestigten Deckel (14), wobei der Deckel (14) in der Lage ist, eine verriegelte Stellung, in der er fest am Körper (16) so verriegelt ist, dass das Innere der Kartusche (10) dicht verschlossen ist, eine geschlossene Stellung, in der er mit dem Körper (16) so zusammenwirkt, dass das Innere der Kartusche (10) noch dicht verschlossen, aber entriegelt ist, und eine offene Stellung einzunehmen, in der ein Zugang zum Inneren der Kartusche (10) möglich ist, wobei der Körper (16) mit einem Element (22) zum Zusammenwirken mit einem Gehäuse (40) des Duftstoffdiffusors (12) versehen ist und wobei der Deckel (14) mit einem Element (20) zum Zusammenwirken mit dem Gehäuse (40) versehen ist, wobei eine Dichtung vorgesehen ist, die das Innere der Kartusche (10) sowohl in der verriegelten als auch in der geschlossenen Stellung des Deckels (14) dicht verschließt, **dadurch gekennzeichnet, dass** zwischen dem Körper (16) und dem Deckel (14) ein Filmscharnier (18) vorgesehen ist.

2. Kartusche nach Anspruch 1, wobei ein Rastmechanismus vorgesehen ist, der dazu eingerichtet ist, den Deckel (14) mit dem Körper (16) zu verriegeln.

3. Kartusche nach einem der vorhergehenden Ansprüche, wobei das Element (22) des Körpers (16) ein Vorsprung zum Zusammenwirken mit dem Gehäuse (40) eines Duftstoffdiffusors (12) ist.

4. Kartusche nach einem der vorhergehenden Ansprüche, wobei das Element (20) des Deckels (14) ein Vorsprung zum Zusammenwirken mit dem Gehäuse (40) eines Duftstoffdiffusors (12) ist.

5. Kartusche nach einem der vorhergehenden Ansprüche, wobei der Deckel (14) mit einem Eingriffselement (26) zum Zusammenwirken mit einer Betätigungsvorrichtung versehen ist.

6. Duftstoffdiffusor, insbesondere für Fahrzeuge, mit einer Kartusche (10) nach einem der vorhergehenden Ansprüche und mit einem Aufnahmeraum (42) für die Kartusche (10) und einer Betätigungsvorrichtung zum Öffnen des Deckels (14) der Kartusche (10), wobei die Betätigungsvorrichtung eine Vorrichtung zum Überführen der Kartusche (10) aus ihrem verriegelten Zustand in ihren geschlossenen gebrauchsfertigen Zustand im Duftstoffdiffusor (12) umfasst, **dadurch gekennzeichnet, dass** die Betätigungsvorrichtung zwei Zusammenwirkungselemente zum Eingriff mit dem Deckel (14) bzw. dem Körper (16) der Kartusche (10) umfasst.

7. Duftstoffdiffusor nach Anspruch 6, wobei die Betätigungsvorrichtung einen Schrittmotor (32) umfasst.

8. Duftstoffdiffusor nach Anspruch 6 oder 7, wobei die Betätigungsvorrichtung ein Betätigungselement (50) zum Zusammenwirken mit dem Deckel (14) der Kartusche (10) umfasst.

9. Duftstoffdiffusor nach Anspruch 8, wobei das Betätigungselement (50) ein Stift ist.

10. Duftstoffdiffusor nach einem der Ansprüche 6 bis 9, wobei die Zusammenwirkungselemente als Nuten (54, 56) ausgebildet sind, in die am Kartuschenkörper (16) und am Kartuschendeckel (14) ausgebildete Elemente eingreifen können.

11. Verfahren zum Überführen einer Duftstoffkartusche (10) nach einem der Ansprüche 1 bis 5 aus einem Ausgangszustand in einen Gebrauchszustand, wobei die Kartusche (10), die einen Körper (16) und einen Deckel (14) aufweist, in einen Duftstoffdiffusor (12) eingesetzt wird und wobei während des Einsetzens der Deckel (14) automatisch aus einer verriegelten Stellung, in der das Innere der Kartusche (10) mittels des Deckels (14) hermetisch verschlossen ist, in eine geschlossene Stellung überführt wird, in der der Deckel (14) das Innere des Körpers (16) noch dicht verschließt, aber nicht mehr mit dem Körper (16) verriegelt ist.

## Revendications

1. Cartouche (10) pour un diffuseur de parfum (12), en particulier pour véhicules, comprenant un corps (16) et un couvercle (14) fixé au corps (16), le couvercle (14) étant apte à prendre une position verrouillée dans laquelle il est verrouillé de manière solidaire au corps (16) de telle sorte que l'intérieur de la cartouche (10) est étanché, une position fermée dans laquelle il coopère avec le corps (16) de sorte que l'intérieur de la cartouche (10) est toujours étanché mais déverrouillé, et une position ouverte dans laquelle l'accès à l'intérieur de la cartouche (10) est possible, le corps (16) étant pourvu d'un élément (22) pour la coopération avec un boîtier (40) du diffuseur de parfum (12), et le couvercle (14) étant pourvu d'un élément (20) pour la coopération avec le boîtier (40), un joint qui étanche l'intérieur de la cartouche (10) tant dans la position verrouillée que dans la position fermée du couvercle (14) étant prévu, **caractérisée en ce qu'**une charnière à film (18) est prévue entre le corps (16) et le couvercle (14).

2. Cartouche selon la revendication 1, dans laquelle il est prévu un mécanisme d'enclenchement qui est apte à verrouiller le couvercle (14) au corps (16).

3. Cartouche selon l'une des revendications précédentes, dans laquelle l'élément (22) du corps (16) est une saillie pour la coopération avec le boîtier (40) d'un diffuseur de parfum (12).

4. Cartouche selon l'une des revendications précédentes, dans laquelle l'élément (20) du couvercle (14) est une saillie pour la coopération avec le boîtier (40) d'un diffuseur de parfum (12).

5. Cartouche selon l'une des revendications précédentes, dans laquelle le couvercle (14) est pourvu d'un élément d'engagement (26) pour la coopération avec un dispositif d'actionnement.

6. Diffuseur de parfum, en particulier pour véhicules, comprenant une cartouche (10) selon l'une des revendications précédentes et présentant un espace de réception (42) pour la cartouche (10) et un dispositif d'actionnement pour ouvrir le couvercle (14) de la cartouche (10), le dispositif d'actionnement comprenant un dispositif pour faire passer la cartouche (10) de son état verrouillé à son état fermé et prêt à l'emploi dans le diffuseur de parfum (12), **caractérisé en ce que** le dispositif d'actionnement comprend deux éléments de coopération pour l'engagement du couvercle (14) et du corps (16) de la cartouche (10), respectivement.

7. Diffuseur de parfum selon la revendication 6, dans lequel le dispositif d'actionnement comprend un moteur pas-à-pas (32).

8. Diffuseur de parfum selon la revendication 6 ou 7, dans lequel le dispositif d'actionnement comprend un élément d'actionnement (50) pour la coopération avec le couvercle (14) de la cartouche (10).

9. Diffuseur de parfum selon la revendication 8, dans lequel l'élément d'actionnement (50) est une goupille.

10. Diffuseur de parfum selon l'une des revendications 6 à 9, dans lequel les éléments de coopération sont réalisés sous forme de rainures (54, 56) dans lesquelles des éléments réalisés sur le corps de cartouche (16) et sur le couvercle de cartouche (14) peuvent s'engager.

11. Procédé pour transférer une cartouche de parfum (10) selon l'une des revendications 1 à 5 d'un état initial à un état d'utilisation, dans lequel la cartouche (10) présentant un corps (16) et un couvercle (14) est insérée dans un diffuseur de parfum (12) et dans lequel, pendant l'insertion, le couvercle (14) est automatiquement transféré d'une position verrouillée, dans laquelle l'intérieur de la cartouche (10) est fermé hermétiquement au moyen du couvercle (14), à une position fermée dans laquelle le couvercle (14) étanche toujours l'intérieur du corps (16) mais n'est plus verrouillé au corps (16).
